# FASCICULE DE BREVET EUROPEEN

(11) **EP 1 730 275 B1**
(45) Date de publication et mention de la délivrance du brevet: **19.01.2011**
(21) Numéro de dépôt: 05744634.6
(22) Date de dépôt: 29.03.2005
(51) Int. Cl.: C12N 9/10, C12N 15/54, C12P 19/38, C12N 15/09

(54) **N-DESOXYRIBOSYLTRANSFERASES DE LACTOBACILLUS FERMENTUM ET APPLICATION A LA SYNTHESE ENZYMATIQUE DE 2', 3' -DIDESOXYNUCLEOSIDE ET DE 2',3'-DIDEHYDRO-2', 3'-DIDESOXYNUCLEOSIDES**
LACTOBACILLUS FERMENTUM N-DESOXYRIBOSYL-TRANSFERASEN UND DEREN VERWENDUNG ZUR ENZYMATISCHEN SYNTHESE VON 2',3'-DIDESOXYNUCLEOSIDEN UND 2',3'-DIDEHYDRO-2',3'-DIDESOXYNUCLEOSIDEN
LACTOBACILLUS FERMENTUM N-DESOXYRIBOSYL TRANSFERASES AND THE USE THEREOF FOR ENZYMATIC SYNTHESIS OF 2', 3' -DIDESOXYNUCLEOSIDES AND 2',3'-DIDEHYDRO-2', 3'-DIDESOXYNUCLEOSIDES

(30) Priorité: 30.03.2004 FR 0403319
(43) Date de publication de la demande: 13.12.2006
(73) Titulaire: INSTITUT PASTEUR, 75015 Paris (FR)
(72) Inventeur: KAMINSKI, Pierre-Alexandre, F-75003 Paris (FR)
(74) Mandataire: Caen, Thierry Alain
(86) Numéro de dépôt international: PCT/FR2005/000743
(87) Numéro de publication internationale: WO 2005/095596

(56) Documents cités:
- WO-A-03/025163
- WO-A-2004/087918
- SUTHERLAND JOHN D ET AL: "Directed evolution of novel biosynthetic pathways: Growth of an Escherichia coli proline auxotroph on DELTA-1-pyrroline-2-carboxylic acid" BIOORGANIC AND MEDICINAL CHEMISTRY LETTERS, vol. 3, no. 6, 1993, pages 1185-1188, XP002337070 ISSN: 0960-894X
- CHARTRAIN M ET AL: "METABOLIC ENGINEERING AND DIRECTED EVOLUTION FOR THE PRODUCTION OF PHARMACEUTICALS" CURRENT OPINION IN BIOTECHNOLOGY, LONDON, GB, vol. 11, no. 2, avril 2000 (2000-04), pages 209-214, XP001148060 ISSN: 0958-1669
- BORNSCHEUER W T: "DIRECTED EVOLUTION OF ENZYMES FOR BIOCATALYTIC APPLICATIONS" BIOCATALYSIS AND BIOTRANSFORMATION, HARWOOD ACADEMIC PUBL., BASEL, CH, vol. 19, no. 2, juillet 2001 (2001-07), pages 85-97, XP001018347 ISSN: 1024-2422
- CARSON D.A.; WASSON D.B.: BIOCHEM.BIOPHYS.RES.COMMS., vol. 155, no. 2, 1988, XP002044161
- INSTITUT PASTEUR RAPPORT D'ACTIVITÉ DE L'UNITÉ CHIMIE ORGANIQUE POUR L'ANNEE 2001, [en ligne] 2002, Extrait de l'Internet: <URL:http://www.pasteur.fr/recherche/RAR/RA R2001/Chimorg.html> [extrait le 2008-08-19]

## Description

La présente invention concerne de nouvelles N-désoxyribosyltransférases de *Lactobacillus fermentum* et leur application à la synthèse enzymatique de 2',3'-didésoxynucléosides et de 2',3'-didéhydro-2',3'-didésoxynucléosides.

Les analogues de nucléosides sont très largement utilisés dans les thérapies antivirales ou dans la chimiothérapie anti cancer. On peut citer par exemple la ddl (didanosine), la ddC (zalcitabine) et la d4T (stavudine) ou l'AZT (zidovudine) dans la thérapie anti-VIH, l'ACV (acyclovir) dans le traitement de l'herpès ou encore le GCV (ganciclovir) utilisé dans la thérapie anti tumorale en combinaison avec la thymidine kinase de l'herpès.

Les didésoxynucléosides tels ddl et ddC et leurs dérivés sont les inhibiteurs les plus efficaces connus à ce jour utilisés dans la thérapie contre le virus HIV.

La synthèse chimique de ces composés nécessite plusieurs étapes de protections, déprotections et purifications. Il serait donc souhaitable de pouvoir simplifier les procédures de synthèse de ce type de composés en développant des méthodes enzymatiques sélectives et hautement spécifiques.

Les N-désoxyribosyltransférases produites par les bactéries du genre *Lactobacillus* sont des enzymes qui catalysent le transfert de désoxyribose entre deux bases puriques ou pyrimidiques. Elles sont également capables en général de transférer le 2',3'-didésoxyribose entre ces mêmes bases (Carson et Wasson, 1988). Ainsi, plusieurs pyrazolo (3,4-d) pyrimidines et triazolo (4,5-d) pyrimidines dérivées de la 2',3'-didésoxycytidine et de la base correspondante ont pu être synthétisées à partir d'enzymes de *Lactobacillus leichmannii* et *Lactobacillus helveticus* (Fischer et coll, 1990). La réaction de transfert de 2',3'-didésoxyribose est toutefois nettement moins efficace que celle effectuée avec le 2'-désoxyribose.

Le clonage de gènes codant des enzymes natives N-désoxyribosyltransférases de *lactobacillus fermentum* a été décrit dans la demande WO 03/025163.

Les inventeurs ont décrit également des protéines mutées de *lactobacillus leichmanii* ayant une activité enzymatique NTD différente de celles décrites jusqu'alors.

D'autres documents d'art antérieur (Sutherland J. D. et al. (1993): "Directed evolution of novel biosynthetic pathways : growth of an Escherichia coli proline auxotrop on delta-1-pyrroline-2-carboxylic acid", Bioorganic and Medicinal Chemistry Letters, vol. 3, no. 6, pages 1185-1188; Chartrain M. et al. (2000): "Metabolic engineering and directed evolution for the production of pharmaceuticals", Current Opinion on Biotechnology, London, vol. 11, no. 2, pages 209-214; Bornscheuer W.T. (2001) : " Directed evolution of enzymes for biocatalytic applications", Biocatalysis and Biotransformation, vol. 19, no. 2, pages 85-97) décrivent des méthodes de mutagénèse dirigée utilisées pour modifier l'activité enzymatique de certaines protéines. Cependant, ces méthodes de mutagénèse dirigée ne concernent pas les N-désoxyribosyltransférases. De plus, ces méthodes n'ont pas permis d'élargir le spectre d'activités enzymatiques desdites protéines.

On a trouvé dans le cadre de la présente invention que l'introduction de mutations dans la N-désoxyribosyltransférase de *Lactobacillus fermentum* (*L. fermentum*)*,* suivie d'une confrontation avec un analogue du substrat naturel au sein du criblage sélectif permettait d'obtenir un protéine mutée ayant une forte activité sur le nouveau substrat. En répétant ces opérations, il est apparu possible d'obtenir des enzymes présentant une activité sur des substrats de plus en plus éloignés du substrat naturel initial.

C'est à la suite d'une étape de mutagenèse aléatoire dans un gène *ntd* de *L. fermentum,* suivie d'une étape de sélection utilisant un crible génétique fonctionnel qu'il a été possible d'isoler des mutants ayant une activité spécifique plus importante, notamment pour le transfert de 2',3'-didésoxyribose.

Ce procédé de sélection d'enzymes modifiées plus actives fait plus particulièrement intervenir comme crible génétique la souche *E.coli* PAK 9 (déposée à la CNCM le 27 Juin 2002 sous le numéro d'accession I-2902), laquelle est de génotype *ΔpyrC* ::Gm, *ΔcodA* ::Km, cdd *::Tn10.*

Cette souche permet de sélectionner la production d'uracile car elle est délétée pour le gène *pyrC* qui commande la conversion de carbamyl aspartate en dihydroorotate ainsi que pour les gènes *codA* et *cdd* qui commandent respectivement la désamination de la cytosine et de la (désoxy)cytidine. Elle présente donc une exigence en uracile (u) qui ne peut être satisfaite que par l'apport d'uridine (R-U), de désoxyuracile (dR-U) ou de didésoxyuracile (ddR-U). Cependant l'utilisation de didésoxyuracile (ddR-U) peut être sélectionnée dans la souche PAK9 uniquement si un variant de la N-désoxyribosyltransférase est capable de réaliser une des deux réactions suivantes:
i) ddR-U →U + ddR,
ii) ddR-U + C ⇄ ddR-C+U.

Les clones transformants de PAK 9 exprimant un gène *ntd* de *L*. *fermentum* muté aléatoirement, ont ainsi été sélectionnés en milieu minéral glucose additionné de didésoxyuracile (ddR-U) et de cytosine (C). Plusieurs transformants ont été obtenus et sont capables de réaliser l'échange

ddR-Pyr + Pur ⇄ ddR-Pur + Pyr

ainsi que

dR-Pyr + Pur ⇄ dR-Pur + Pyr.

Les séquences nucléotidiques des différents variants de *ntd* de *L. fermentum* peuvent différer du gène sauvage que d'une seule mutation. Leurs activités enzymatiques ont été évaluées à partir d'extraits bruts ou des protéines purifiées. L'activité spécifique de NTD* peut être 10 fois inférieure à celle de NTD pour le transfert de désoxyribose mais peut être 7 fois supérieure pour le transfert de didésoxyribose.

L'enzyme sélectionnée trouve son application dans la synthèse enzymatique de 2',3'-didésoxynucléoides et de 2',3'-didéhydro-2',3'-didésoxynucléosides de bases naturelles ou modifiées (5-halogéno-pyrimidines), comportant ou non des radioéléments. Le procédé peut être étendu à la sélection de variants capables de transférer des dérivés de 2'-désoxyribose ou 2',3'-didésoxyribose entre bases (tel que, 3'-amino-2',3'-didésoxyribose ou 3'-azido-2',3'-didésoxyribose).

La présente invention a plus particulièrement pour objet une protéine isolée ayant une telle activité N-didésoxyribosyltransférase, présentant un pourcentage d'identité avec SEQ ID NO. 4 égal ou supérieur à 70% et comprenant un résidu thréonine correspondant au point de mutation A15T.

### DESCRIPTION

Les inventeurs ont mis au point un procédé général d'évolution artificielle *in vitro* et *in vivo* d'une protéine X codée par un gène *ntd* de *L. fermentum,* ledit procédé permettant de faire évoluer *in vivo* ladite protéine X par complémentation soit d'une protéine apparentée, soit par complémentation d'une voie métabolique inactivée.

Un tel procédé permet de faire évoluer une protéine X codée par un gène *ntd* de *L. fermentum* de sorte à en modifier ses caractéristiques par les étapes suivantes :
a) obtention de mutants du gène *ntd* de *L. fermentum* par mutagenèse aléatoire ;
b) transformation de cellules comportant un phénotype [P-] avec des vecteurs comportant les acides nucléiques mutés obtenus à l'étape a) codant pour les protéines ainsi modifiées X*, P- signifiant que lesdites cellules sont auxotrophes pour la substance P, P étant le produit de l'action de X sur son substrat naturel S ;
c) mise en culture desdites cellules dans un milieu comprenant un substrat S*, S* étant un analogue du substrat naturel S de ladite protéine X;
d) sélection des cellules [P- :: X*] qui ont survécu à l'étape c) dans lesquelles les protéines X* sont capables de réaliser la biosynthèse du produit P à partir du substrat S*.

La protéine mutante X* obtenue est une protéine possédant une activité voisine de celle de la N-désoxyribosyltransférase naturelle X. X* appartient ainsi à des classes enzymatiques communes ou voisines des N-désoxyribosyltransférases présentant au moins les trois premiers chiffres des classes EC de la nomenclature internationale à 4 chiffres. Pour le passage d'une classe à une autre, on peut répéter le procédé mentionné ci-dessus avec à chaque passage l'addition d'une modification supplémentaire sur l'analogue de substrat désigné par S*.

Par "analogue de substrat", on entend le substrat S naturel de la protéine X naturelle comportant une modification ou une altération. Par "modification de ce substrat", on entend l'addition ou la suppression d'au moins un atome, un groupe ou substituant, la modification de la conformation spatiale du substrat (isomère, énantiomère, diastéroisomère). Cette modification peut être minime ou importante du point de vue structurel. Dans le cas où on cherche à modifier de manière substantielle l'activité de la protéine (ou enzyme), on peut procéder par répétition du procédé en modifiant d'avantage le substrat S* à chaque nouveau cycle de sélection. Peu à peu, la protéine accumule des mutations qui sont responsables de la modification de son activité.

Dans ce procédé, les cellules utilisées à l'étape b) sont obtenues par inactivation d'un moins un gène impliqué dans la voie métabolique naturelle conduisant au produit P.

Ainsi, la protéine X* obtenue complémente la déficience de la voie métabolique naturelle conduisant au produit P dans un milieu pourvu du substrat S*.

Par "complément", on entend la suppression du phénotype auxotrophe résultant de l'inactivation du gène ou de la voie métabolique.

Alternativement, les cellules peuvent être des cellules dans lesquelles le gène codant pour une protéine apparentée à X a été inactivé au préalable.

Par "inactivation", on entend une délétion en tout ou en partie, une insertion, ou une mutation rendant inopérant le gène. L'inactivation peut également consister en une modification conduisant à un phénotype du type Ts (température sensible). Dans ce cas, les cellules sont cultivées à des températures non permissibles pendant la phase de sélection (étapes c) et d)).

De préférence, la protéine apparentée Y précédemment citée possède au moins les trois premiers chiffres (2.4.2) de la nomenclature internationale EC à 4 chiffres (tableau 1), plus particulièrement fait partie de la classe EC 2.4.2.6 (N-désoxyribosyltransférases).

**TABLEAU 1**

| **Numéro EC** | **Nom selon la nomenclature internationale** |
|---|---|
| 2.4.2.5 | Nucleoside ribosyltransférase. |
| 2.4.2.6 | Nucléoside déoxyribosyltransférase. |
| 2.4.2.7 | Adenine phosphoribosyltransférase |
| 2.4.2.8 | Hypoxanthine phosphoribosyltransférase. |
| 2.4.2.9 | Uracil phosphoribosyltransférase. |
| 2.4.2.10 | Orotate phosphoribosyltransférase. |
| 2.4.2.11 | Nicotinate phosphoribosyltransférase. |
| 2.4.2.12 | Nicotinamide phosphoribosyltransférase. |
| 2.4.2.14 | Amidophosphoribosyltransférase. |
| 2.4.2.17 | ATP phosphoribosyltransférase. |
| 2.4.2.18 | Anthranilate phosphoribosyltransférase. |
| 2.4.2.20 | Dioxotetrahydropyrimidine phosphoribosyltransférase. |
| 2.4.2.21 | Nicotinate-Nucléotide-dimethylbenzimidazole phosphoribosyltransférase. |
| 2.4.2.22 | Xanthine-guanine phosphoribosyltransférase |
| 2.4.2.29 | Queuine tRNA-ribosyltransférase. |
| 2.4.2.30 | NAD(+) ADP-ribosyltransférase. |
| 2.4.2.31 | NAD(P)(+)--arginine ADP-ribosyltransférase. |
| 2.4.2.36 | NAD(+)-diphthamide ADP-ribosyltransférase. |
| 2.4.2.37 | NAD(+)-dinitrogeN-reductase ADP-D-ribosyltransférase. |

Avantageusement, l'activité de la N-désoxyribosyltransférase X sur le substrat S est au moins 2, 5, 10, 25, 50, 100 ou 1000 fois supérieure à son activité sur le substrat S*. Parallèlement, l'activité de la protéine X* sur le substrat S* est au moins 5, 10, 25, 50, 100 ou 1000 fois supérieure à son activité sur le substrat S.

La mutagénèse aléatoire de l'étape a) peut être effectuée soit par variation de la concentration en manganèse lors de la réaction PCR, soit par utilisation d'analogues de nucléotides promutagènes ou encore par la mise en oeuvre d'amorces comprenant une séquence aléatoire. Différentes techniques sont décrites dans les documents US 6,323,030 (Methods for generating polynucleotides having desired characteristics by iterative selection and recombination), US 6,177,263 (Recombination of polynucleotide sequences using random or defined primers), WO 01/66798 (Random truncation and amplification of nucleic acid), and EP1205547 (DNA mutagenesis by random fragmentation and reassembly).

Les cellules utilisées dans le cadre de l'invention sont des cellules procaryotes ou eucaryotes, de préférence *E. coli.*

Dans un mode de réalisation particulier de ce procédé, a fait évoluer une N-désoxyribosyltransférase (DTP) de sorte à obtenir une N-didésoxyribosyltransférase, caractérisé en ce qu'il comprend les étapes suivantes :
a) obtention de mutants DTP* de la séquence du gène *ntd* de *L*. *fermentum* codant pour une N-désoxyribosyltransférase (DTP) par mutagénèse aléatoire ;
b) transformation de cellules comportant un phénotype [N-] avec des vecteurs comportant les acides nucléiques mutés obtenus à l'étape a) codant pour les protéines DTP*, N- signifiant que lesdites cellules sont auxotrophes pour au moins un nucléoside, ledit nucléoside étant le produit de l'action de DTP sur son substrat naturel dR-N ;
c) mise en culture desdites cellules dans un milieu comprenant un substrat ddR-N;
d) sélection des cellules [N- :: DTP*] qui ont survécu à l'étape c) dans lesquelles les protéines DTP* sont capables de réaliser le transfert du didésoxyribose (ddR) d'un didésoxyribonucléoside à un autre nucléoside conduisant à la production du nucléoside N nécessaire pour la survie des cellules.

Par "nucléoside N", on entend un nucléoside naturel, c'est à dire des molécules constituées d'un sucre relié à une base hétérocyclique par une liaison N-glycosidique, les bases étant des pyrimidines (thymine, uracile, cytosine) ou des purines (adénine, guanine parmi les bases usuelles). Par "N- ", on entend un phénotype [A-, T-, G-, C-, U- ou I-].

L'enzyme NTD* obtenu peut être capable de reconnaître et transférer un analogue de désoxyribose tel que le didésoxyribose, mais également d'agir sur des analogues de nucléoside. Ainsi, l'analogue de substrat S* utilisé peut être un analogue de désoxyribonucléoside ou didéhydrodidésoxyribonucléosides comportant au moins une modification chimique sur la base et/ou sur le ribose.

Plus particulièrement, la séquence codante (*ntd*) de la N-désoxyribosyltransférase (DTP) de *L. fermentum* correspond à SEQ ID No 1.

Dans ce procédé, on peut utiliser à l'étape b) des bactéries de génotype Δ*pyrC*,*ΔcodA,* Δ*cdd* déficientes dans la voie métabolique conduisant à l'uracile. La souche d'*E.coli* PAK 9 déposée à la CNCM le 27 Juin 2002 sous le N° I-2902, est particulièrement adaptée à cet usage.

La présente invention vise, à partir du procédé décrit ci-dessus, à obtenir à partir de la protéine X codée par *ntd* de *L. fermentum,* une protéine mutée présentant une activité N-didésoxyribosyltransférase et/ou une activité sur des analogues de désoxy ou didésoxyribonucléoside comportant une base modifiée. La séquence de la protéine ainsi mutée présente en général un pourcentage d'identité supérieur ou égal à 70 %, notamment 80 %, préférentiellement supérieur ou égal à 90 %, et plus préférentiellement supérieur ou égal à 95 % avec la séquence SEQ ID No.2. Il est en outre important que certains résidus de la séquence ID No.2 soient conservés pour que ladite protéine mutée présente une activité enzymatique optimale. C'est le cas en particulier des résidus Y13 (tyrosine en position 13), D77 (acide aspartique en position 77), D97 (acide aspartique en position 97), E103 (acide glutamique en position 103), M132 (méthionine en position 132). Ainsi, certains variants peuvent présenter un pourcentage d'identité avec la séquence ID No. 2 compris entre 70 % et 80 % dans les régions qui sont situées en dehors du site catalytique de l'enzyme constitué par lesdits résidus. Ces variants présentent alors une séquence identique au moins à 70 % à SEQ ID No.2, dans laquelle les résidus Y13, D77, D97, E103, M132 sont conservés, de préférence au moins à 80 %.

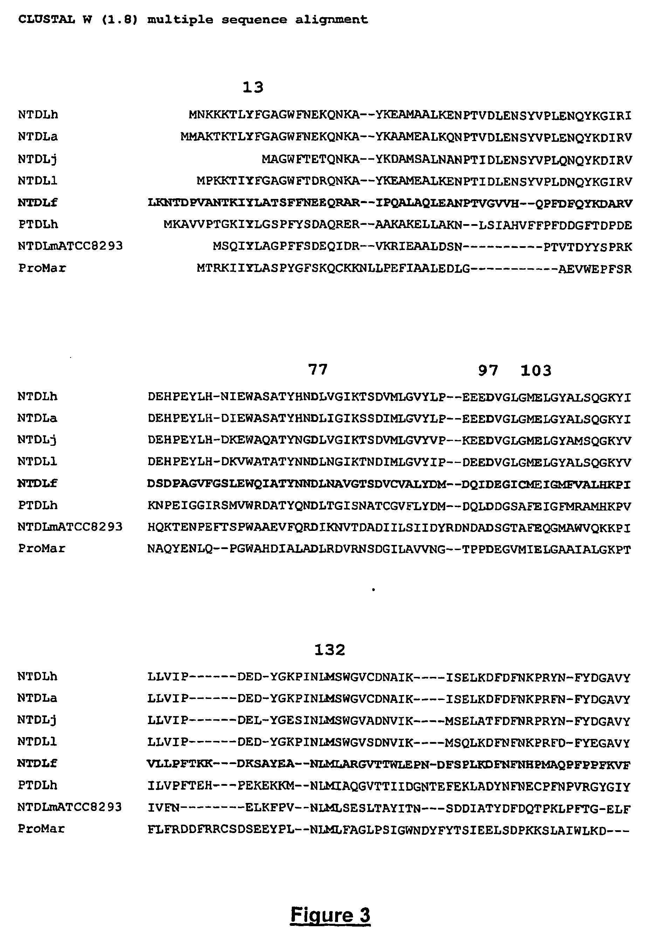

L'invention consiste ainsi également en une protéine ayant une activité sur des analogues de désoxy- ou didésoxyribonucléosides, présentant un pourcentage d'identité avec SEQ ID N°4 égale ou supérieure à 70 %, de préférence 75 %, et dans un ordre préférentiel, respectivement 80 %, 85 %, 90 %, 95 % et 98 %, et comprenant un résidu thréonine correspondant au point de mutation A15T de SEQ ID N°4. La correspondance entre le résidu thréonine et le point de mutation A15T de SEQ ID N°4 est en général établi par alignement de la séquence de ladite protéine avec SEQ ID N°4 comme cela est représenté dans la figure 3 de la présente demande.

Une telle protéine comprend en général, en outre, les résidus correspondants à Y13, D77, D97, E103 et M132 de SEQ ID N°4, qui sont nécessaires à une bonne activité catalytique.

De préférence, une protéine selon l'invention présente une activité N-didésoxyribosyltransférase, ce qui se traduit en général par une activité de transfert du désoxyribose et du didésoxyribose et/ou didéhydroribose.

Une protéine telle que définie ci-dessus présente en général une activité catalytique sur d4T et ddT supérieure, de préférence au moins 50 %,à celle de la protéine N-désoxyribosyltransférase native de *L.fermentum* représentée par SEQ ID N°2.

Cette activité catalytique se traduit notamment par une efficacité catalytique sur d4T et ddT au moins 5 fois, de préférence au moins 7 fois, supérieure à celle de la protéine N-désoxyribosyltransférase native de *L.fermentum* représentée par SEQ ID N°2. L'efficacité catalytique sur ddT est en général 10 fois, de préférence 20 fois et plus préférentiellement 50 fois supérieure à celle de la protéine N-désoxyribosyltransférase native de *L.fermentum* représentée par SEQ ID N°2.

On entend par efficacité catalytique le résultat du quotient Kcat/Km, qui reflète le nombre de fois qu'une enzyme effectue une réaction (transformation de son substrat), comparé au nombre de fois que ladite enzyme forme un complexe avec son substrat. Ainsi plus une enzyme est efficace, plus élevée sera la valeur de son quotient Kcat/Km.

Une protéine mutée particulièrement préférée de l'invention comprend la mutation A15T, comme par exemple la protéine de séquence SEQ ID No 4.

L'invention concerne également un acide nucléique comprenant une séquence de *ntd* mutée (NTD*) codant pour une protéine mutée telle que définie précédemment et ayant une activité N-désoxyribosyltransférase et/ou une activité sur des analogues de désoxy ou didésoxyribonucléoside comportant une base modifiée mutée. Un acide nucléique préféré de l'invention comprend la séquence SEQ ID No 3, lequel code pour la protéine correspondant à SEQ ID No.4.

L'invention porte également sur un vecteur d'expression comprenant un acide nucléique tel que défini ci-dessus, en particulier la séquence SEQ ID No.3. Cette séquence peut être fusionnée à un promoteur efficace pour l'expression de toute ou partie de ladite séquence dans les cellules eucaryotes et/ou procaryotes. Le vecteur peut être un plasmide capable de transformer et de se maintenir chez *E. coli.* Le vecteur peut se maintenir dans la bactérie de manière stable ou transitoire.

L'invention vise également une cellule hôte comprenant un vecteur tel que décrit précédemment, telle que la souche d'*E*. *coli* déposée à la -CNCM le 22 mars 2004 sous le numéro d'accession I-3192 qui comprend le vecteur pETLFA15T décrit plus loin.

Dans un autre aspect, l'invention se rapporte à l'utilisation d'une N-didésoxyribosyltransférase décrite ci-dessus pour le transfert d'un didéoxyribose (ddR) d'un didésoxyribonucléoside sur un autre nucléoside, en particulier dans le but d'obtenir la synthèse de 2',3'-didesoxynucléosides et de 2',3'-didéhydro-2',3'-didesoxynucléosides.

Cet enzyme obtenue à partir du procédé selon l'invention est particulièrement utile pour la préparation d'analogues de nucléosides possédant des propriétés antitumorales, notamment du ddl ou ddC.

Ainsi, l'invention porte également sur un procédé de préparation de composés comprenant une étape consistant à mettre en oeuvre une protéine mutée définie ci-dessus.

Ce procédé est particulièrement avantageux pour la préparation d'analogues de nucléosides ou nucléotides utiles pour le traitement du cancer ou de maladies infectieuses, notamment des didésoxyribonucléosides, en particulier le ddC ou ddl et de 2',3'-didéhydro-2',3'-didésoxynucléosides.

On se référera aux légendes des figures ci-après pour la suite de la description.

### Légendes

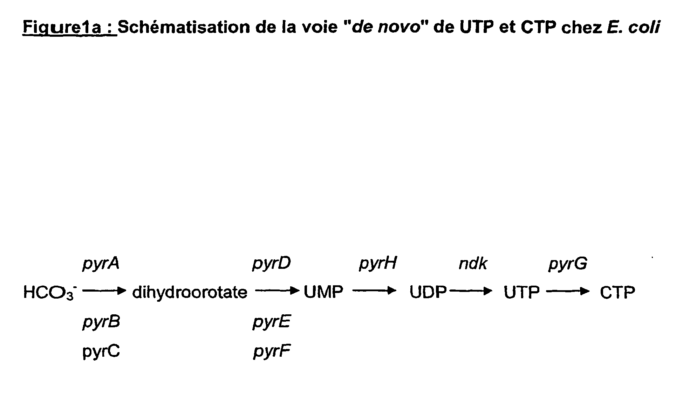

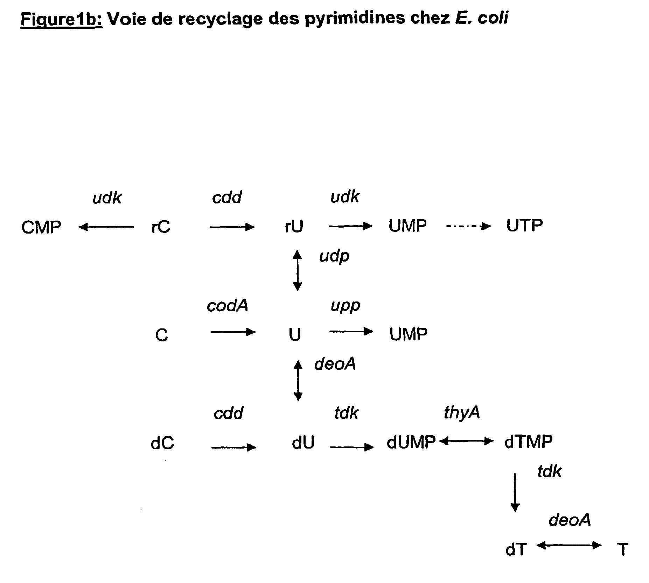

- Figure 1 : Voies de biosynthèses
- figure 1 a) la synthèse "*de novo"* de l'ADN à partir de précurseurs simples. Les abréviations utilisées sont les suivants :
   *ndk*: nucléoside diphosphokinase
   *pyrA:* carbamoylphosphate synthase
   *pyrB:* aspartate carbamoyltransférase
   *pyrC:* dihydroorotase
   *pyrD:* dihydroorotate oxydase
   *pyrE:* orotate phosphoribosyltransférase
   *pyrF:* orotidine 5'-phosphate decarboxylase
   *pyrG:* CTP synthétase
   *pyrH:* UMP kinase
- figure 1 b) la voie de sauvegarde ou de recyclage bien moins coûteuse en énergie et impliquant des réactions de transfert de sucre à partir de bases préformées (issues de la dégradation hydrolytique d'acides aminés et de nucléotides). Les abréviations utilisées sont les suivantes (enzymes représentées par leurs gènes correspondants) :
   *cdd*: cytidine/désoxycitidine désaminase
   *cmk:* CMP/dCMP kinase horylase
   *codA*: cytosine désaminase
   *deoA*: thymidine phosphorylase
   *tdk*: thymidine kinase
   *udk:* uridine/cytidine kinase
   *udp:* uridine phosphorylase
   *upp:* uridine phosphoryltransferase
   *thyA:* thymidylate synthase
- Figure 2 : Cycle catalytique de NTD
- Figure 3 : Alignement de séquences de Ntd montrant les résidus Y(Tyr)13, D(Asp)77, D(Asp)97, E(Glu)103 et M (Met)132 faisant partie du site catalytique. *Lh : lactobacillus helveticus;* La : *lactobacillus acidophilus,* Lj : *lactobacillus johnsonni,* LI *: lactobacillus leichmanni,* **Lf** : ***lactobacillus fementum,*** Lm : *leuconostoc mesenteroides,* Pro mar : *prochlorococcus marinus*

### EXEMPLE 1 : Synthèse enzymatique de nucléosides

La synthèse des nucléosides chez *E. coli* peut se faire selon deux procédés; [Agnete MUNCH-PETERSEN (1983). "Metabolism of nucleotides, nucleosides and nucleobases in microorganisms" published by Academic Press] (voir figures 1a et 1b).

Il existe deux classes d'enzymes qui catalysent le transfert d'un 2-désoxyribosyle vers une base azotée ; voir ci-après et [Jane R. HANRAHAN & David W. HUTCHINSON (1992). "The enzymatic synthesis of antiviral agents". Journal of Biotechnology; vol.23; 193-210. Celles-ci sont parfois employées pour la synthèse d'analogues de nucléosides].

Les N-désoxyribosyltransférases catalysent le clivage des liaisons glycosidiques des 2-désoxynucléotides. Elles sont présentes chez certains micro-organismes qui ne possèdent pas ou peu de purine et de pyrimidine phosphorylase (lactobacilles par exemple) [6-8]. Elles participent au recyclage des nucléotides.

### Réactions catalysées selon le type d'enzymes

Deux types d'enzyme ont été caractérisés, [José HOLGUIN & Robert CARDINAUD (1975). "Trans-N-Deoxyribosylase: substrate specific studies". European Journal of Biochemmistry; vol.54 ; 515-520].

### Purine désoxyribosyltransférase ou NTDI:

Elle catalyse exclusivement le transfert réversible d'un sucre d'une base purique (base donneuse) vers une autre purine (base receveuse).

(1) : (d)R-pur₁ + pur₂ ⇄ pur₁ + (d)R-pur₂

### Pyrimidine/Purine désoxyribosyltransférase ou NTD II :

Elle catalyse majoritairement le transfert entre purine et pyrimidine selon les équations réversibles suivantes :

(2): (d)R-pyr₁ + pyr₂ ⇄ pyr₁ + (d)R-pyr₂

(3): (d)R-pyr₁ + pur₂ ⇄ pyr₁ + (d)R-pur₂

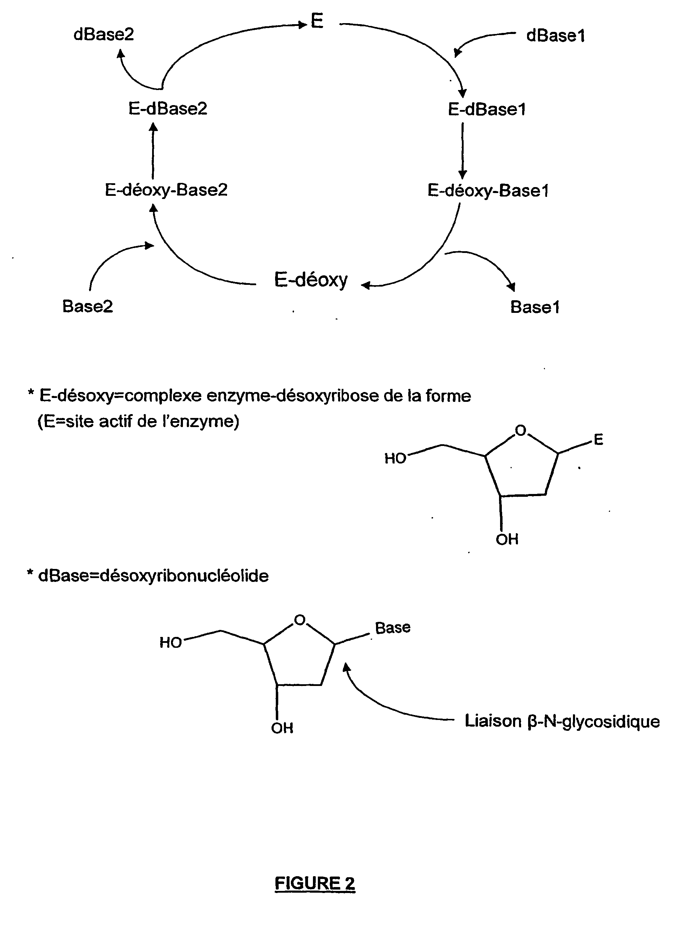

### Mécanisme réactionnel (figure 2)

Si on s'en tient à ce qui est connu chez *Lactobacillus delbruckii,* NTD II de réagirait selon un mécanisme "ping-pong-bi-bi" qui ferait intervenir deux substrats et deux produits [José HOLGUIN & Robert CARDINAUD (1975). "Trans-N-Deoxyribosylase: Purification by affinity chromatography and characterisation". European Journal of Biochemmistry; vol.54; 505-514 ; C. DANZIN & Robert CARDINAUD (1974). "Deoxyribosyl transfer catalysis with trans-N-deoxyribosylase. Kinetic studies of purine to purine trans-N-deoxyribosylase. European Journal of Biochemistry; vol. 48; 255-252 ; C. DANZIN & Robert CARDINAUD (1976). "Deoxyribosyl transfer catalysis with trans-N-deoxyribosylase. Kinetic studies of purine (pyrimidine) to purine (pyrimidine) trans-N-deoxyribosylase. European Journal of Biochemistry; vol.62; 356-372].

On suppose que le sucre du nucléoside donneur (dBase₁) se lie de façon covalente à l'enzyme. Une réaction intramoléculaire au sein de ce complexe binaire permet le clivage de la liaison (β-glycosidique et la formation d'un complexe ternaire E-désoxyribosyl-Base₁ suivie de la libération du premier produit (Base₁). La base acceptrice (Base₂) se fixe alors sur l'intermédiaire binaire et après réaction intramoléculaire sur le site actif de l'enzyme, le second produit (dBase₂) est libéré. L'enzyme peut dès lors mener une autre catalyse.

### Propriétés physico-chimiques

Chez *Lactobacillus delbruckii,* les deux enzymes ont un poids moléculaire voisin (évalué aux alentours de 100 kDa) mais elles diffèrent par leur stabilité thermique (activité observée jusqu'à 45°C pour NTD I et 55°C pour NTD II) et leur pH optimum (6.4 pour NTD I et 6.0 pour NTD II).

Le gène *ntd* de *Lactobacillus delbruckii* codant pour NTD II d'une longueur de 471 bp code pour la synthèse d'une protéine de 157 acides aminés et de poids moléculaire total de 110 kDa [William J.COOK, Steven A. SHORT & Steven E. EALICK (1990). "Crystallization & preliminary X-ray investigation of recombinant Lactobacillus leichmanii nucleoside 2-deoxyribosyltransférase". The Journal of Biological Chemistry; vol.265; No.5; 2682-2683]. La structure cristalline de l'enzyme NTD II de L. delbruckii a été déterminée avec une résolution de 2,5 Å. C'est un hexamère (trimère de dimères) constitué de six sous-unités identiques de 18 kDa. Chaque sous-unité possède au centre un feuillet βparallèle composé de cinq brins de longueurs diverses et entouré par quatre hélices α disposées de façon asymétrique. Chacune comprend un site actif, mais les six centres catalytiques, distants deux à deux d'environ 20 Å, requièrent la participation des chaînes latérales des sous-unités voisines [Shelly R. ARMSTRONG, William J.COOK, Steven A. SHORT & Steven E. EALICK (1996). "Crystal structures of nucleoside 2-deoxyribosyltransférase in native & ligand-bound forms reval architecture of the active site". Structure; vol.4; No.1; 97-107]. Ces dernières sont impliquées dans le positionnement de l'acide aminé catalytique (le glutamate 98) [ David J.T. PORTER, Barbara M. MERRIL & Steven A. SHORT (1995). "Identification of the active site nucleophile nucleoside 2-deoxyribosyltransférase as glutamic acid 98". The Journal of Biological chemistry; vol.270; No.26; 15551-15556].

### Synthèse enzymatique d'analogues de nucléosides

Les réactions de transfert, hautement stéréospécifiques, produisent en présence d'une transférase NTD I ou NTD II, exclusivement l'anomère β du nucléoside (ce qui évite l'étape de séparation des isomères α et β).

L'enzyme possède une grande spécificité vis-à-vis des 2'-désoxyribonucléotides mais tolère un grand nombre d'analogues modifiés sur le sucre ou sur la base. La thymidine et la cytosine semblent les plus efficaces donneurs de sucre. D'autre part le transfert peut s'effectuer sur un large panel de bases receveuses. Citons par exemple les purines substituées en position 6 [D. BETBEDER, D.W. HUTCHINSON & A.O. RICHARDS (1989). "The stereoselective enzymatic synthesis of 9-β-D-2',3'-dideoxynucleosides of N(6)-substitued purines". Antiviral Chem. Chemother ; vol.17; 4217-4222] et dYTP.

### dYTP:

L'imidazole-4-carboxamide noté Y a été proposé comme purine simplifiée. Cet analogue a pour formule:

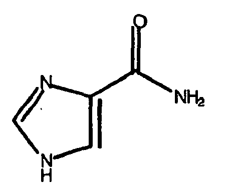

Il a été rapporté que le nucléotide dYTP pouvait se substituer à dATP ou dGTP lors de la copie de l'ADN ce qui introduit des mutations. On peut également citer les composés décrits WO 01/96354 (Institut Pasteur) de formule générale :

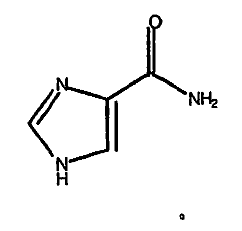

Les enzymes NTD se révèlent capables de catalyser de façon marginale la réaction d'échange entre un 2',3'-didésoxyribose et une base acceptrice :

dd-1'-Base₁ + Base₂ ⇄ Base₁ + dd-1'-Base₂

(dd = 2',3'-didéoxyribose)

Néanmoins la vitesse de ce transfert reste très faible comparée à celle caractérisant l'échange de désoxyriboses.

Les 2',3'-didésoxyribonucléotides présentent un intérêt évident en tant que terminateurs de chaîne dans les procédures de séquençage. De plus la 2',3'-didésoxyadénosine (ddA) et la 2',3'-didésoxyinosine (ddl) sont utilisés à des fins thérapeutiques notamment dans le cas du virus du SIDA: ces analogues inhibent de manière efficace la réplication du VIH (virus d'immunodéficience humaine) [H. MITSUYA & S. BRODER (1987). "Strategies for antiviral therapy in AIDS". Nature ; vol. 325 ; 773-778].

A cette fin, l'invention apporte un nouveau procédé d'obtention de mutants de l'enzyme NTD Il afin de sélectionner des enzymes mutantes de *L. fermentum* qui ont une plus forte spécificité envers les 2',3'-didésoxyribonucléosides que l'enzyme native.

### EXEMPLE 2 :

### Application du procédé selon l'invention pour l'obtention de NTD*

### MATERIELS ET METHODES

Les souches d'*E. coli* PAK9 sont cultivées en milieu Luria-Bertani (LB) ou en milieu minimum MS (Richaud et coll 1993). Les antibiotiques kanamycine, Km, chloramphénicol Cm, sont utilisés à la concentration finale de 25µg/ml; tetracycline, Tc et gentamycine, Gm, 10µg/mL. Les nucleosides et bases sont utilisés dans les milieux de culture à la concentration finale de 0,3mM. Les techniques de biologie moléculaire ont été faites selon Sambrook et coll (1989)

Les produits d'amplification sont purifiés à l'aide du QlAquick PCR purification (QIAgen)

Les fragments d'ADN purifiés sur gel d'agarose sont extraits à l'aide du Kit Jetsorb (Genomed) ou du kit QIAquick gel extraction (QlAgen). L'ADN plasmidique est purifié à l'aide du kit QlAprep spin miniprep (QlAgen)

La souche PAK9 (MG1655 *ΔpyrC*::Gm, Δ*codA*::Km, *cdd::Tn10*) est disponible auprès de la CNCM (Collection Nationale de Culture des Microorganismes) à l'Institut Pasteur, 25-28 rue du Dr Roux 75224 Paris cedex 15, sous le N° I-2902.

Le vecteur pSU19N a été obtenu par mutagénèse dirigée du plasmide pSU19 [B. BARTOLOME, J. JUBETE, E. MARTINEZ & F. DE LA CRUZ (1991) " constructions and properties of a family of pACYC184-derived cloning vectors compatible with pBR322 and its derivatives" Gene; vol.102; 75-78; E. MARTINEZ, B. BARTOLOME & F. DE LA CRUZ (1988) "pACYC184-derived cloning vectors containing the multiple cloning site and lacZ alpha reporter gene of pUC8/9 and pUC18/19 plasmids" gene; vol 68(1); 159-162] à l'aide des oligonucléotides
**PAK 23** 5'P-CAATTTCACACAGGAAACACATATGACCATGATTACGCC (SEQ. ID N° 5)
**PAK 24** 5'P-TGTTTCCTGTGTGAAATTGTTATCCGCTCAC (SEQ. ID N°6)

Un gène *ntd* de *L. fermentum* a été amplifié par PCR à partir du plasmide pLF6 utilisé ici en tant que matrice d'ADN. Le plasmide pLF6 propagé à partir de la souche *E.coli* PAK6 déposée à la CNCM le 2 mai 2001 sous la référence I-2664, contient un fragment Alu I de 1,36 kb du gène codant la N-désoxyribosyltransférase de type II issu de la souche *L.fermentum* CIP102780T. Pour amplifier ce fragment d'ADN, les oligonucléotides suivants ont été utilisés:
**PAK 5** 5'-GATATACATATGAAAAATACCGACCCAGTTGC (SEQ. ID N°7) et
**PAK 6** 5'-NNGGATCCTTAGGTTAGTTAGAAAACCTTGAATGGTGGG (SEQ. ID N°8),
puis les fragments amplifiés ont été digérés par les enzymes de restriction *BamH*I) et *Nde*I et clonés dans le vecteur pSU19N. Dans cette construction, l'expression de la protéine est sous le contrôle du promoteur lac.

### 1) Mutagènese

Les amorces T7prom (5'-TTAATACGACTCACTATAGGGG) (SEQ. ID N°9) et T7term (5'-GCTAGTTATTGCTCAGCGG) (SEQ. ID N°10) ont été utilisés pour amplifier le gène *ntd* cloné dans le plasmide pET24a (Novagen) selon des conditions standard d'amplification à l'aide du GeneMorph PCR Mutagenesis Kit (Stratagene, USA). Les paramètres d'amplification : 1 cycle de 5' à 95°C, 30 cycles comportant chacun les trois étapes suivantes : 30" à 95°C, 30" à 51,5°C, 1' à 72°C, puis un cycle de 10' à 72°C. Les concentrations d'ADN matrice utilisées : 10ng et 10pg.

### 2) Clonage et sélection

Les produits d'amplification purifiés sont digérés durant 2 heures à 37°C par les enzymes de restriction *BamH*I et *Nde*l. Après migration à 150V durant 45 min, ils sont purifiés par extraction du gel d'agarose à 1 % à l'aide du kit QlAquick gel extraction (QIAgen).

Le plasmide pSU19N est digéré par les mêmes enzymes et purifié selon la même procédure.

Les ligatures réalisées dans un volume de 20 µL comprennent 15 ng des produits d'amplification, 50ng de pSU19 digéré par *BamH*I*-Hind*III*,* 2 µL de tampon de réaction 10x concentré de la T4 ADN ligase et 6U de T4 ADN ligase. La réaction est effectuée à 16°C durant 18 heures.

Les produits de ligature sont ensuite dialysés sur filtre Millipore (0,05 µm; 13 mm) pendant 30 min puis utilisés pour transformer la souche PAK9, préparée selon le protocole décrit par Dower et coll (1987), par électroporation.

1 à 5 µL d'ADN ligaturé mélangés à 50 µL de la souche PAK9 dans une cuvette de 2mm sont soumis à une charge de 2,5 kV. Après une incubation d'une heure à 37°C dans 1 ml de milieu LB supplémenté en uracile (0,3 mM), deux lavages successifs avec 1 ml milieu MS 1x sont effectués.

450 µL de suspension sont étalés sur milieu gélosé minéral glucose supplémenté en Cm, ddU et C. Les boites sont incubées à 37°C durant 4 jours. Les colonies sélectionnées sont ensuite isolées sur le même milieu.

L'ADN plasmidique des colonies isolées est préparé à partir de cultures en milieu LB supplémentées en Cm et U. Le séquençage des plasmides a été effectué par la société MWG-BIOTECH.

Le séquençage des plasmides présents dans les transformants de PAK 9 sélectionnés a permis d'identifier une mutation dans la séquence (*ntd*) ayant pour effet de substituer dans la séquence protéique correspondante (SEQ ID N°2) le résidu A en position 15 par un résidu T (mutation notée A15T).

### 3) Mesure de l'activité enzymatique des extraits bruts des différents mutants

### 3.1 Préparation des extraits bruts

Les précultures sont obtenues après inoculation d'une colonie isolée dans 5 mL de milieu LB contenant Cm et U pour la souche PAK9 suivie d'une nuit d'incubation sous agitation à 37°C.

Le lendemain, 15 mL de milieu LB Cm et U sont inoculés à une DO₆₀₀ = 0,01. Les cultures sont ensuite incubées à 37°C jusqu'à une DO comprise entre 0,8 et 1.

Les cellules sont ensuite centrifugées à 4000 rpm pendant 30 minutes à 4°C, le culot est remis en suspension dans 10 ml de tampon phosphate (Na₂HPO₄ + NaH₂PO₄ à 50 mM (pH=7,5). Après centrifugation, le culot est remis en suspension dans 1 ml du même tampon. Les cellules, conservées dans de la glace, subissent alors trois cycles de 30 s de sonication et 30 s de repos. Après centrifugation à 12000 rpm durant 2x15 minutes à 4°C; les sumageants sont récupérés et stockés à -20°C.

### 3_{.}2 Réaction enzymatique

50 µL d'extrait enzymatique sont additionnés à 200 µL de tampon citrate à 100 mM pH 6,44 en présence de ddU ou dU à 3 mM final et de C à 1 mM final pour la souche PAK9, le tout est incubé à 37°C. L'avancement de la réaction est suivi par CCM (Silice ; éluant : MeOH- CH₂Cl₂ (20/80)). Les produits sont révélés sous UV, et les sucres révélés par le réactif de Zücker La disparition des substrats et la formation des produits ont aussi été quantifiés par analyse en HPLC. Les différents produits sont séparés par HPLC analytique avec une colonne phase inverse (100-5C18) en utilisant un débit de 1 ml/min et un gradient linéaire 5-25% CH₃CN dans un tampon triethylammonium acetate 10mM à pH 7,5 pendant 20 min.

### 4) Surproduction et purification de la N-désoxyribosyltransférase native et du mutant LFA15T.

Les oligonucléotides :
**PAK 5** 5'-NGATATACATATGAAAAATACCGACCCAGTTGC (SEQ. ID N°11) et
**PAK 6** 5'-NNGGATCCTTAGGTTAGTTAGAAAACCTTGAATGGTGGG (SEQ. ID N°12)
ont été utilisés comme amorce dans une réaction d'amplification dans des conditions standard en utilisant le gène *ntd* de *L*. *fermentum* cloné dans le pSU19 (pLF6) comme ADN matrice. Le produit d'amplification a été digéré par les enzymes de restriction *Nde*I et *Bam*HI pendant 2h à 37°C, purifié sur gel d'agarose et inséré dans le plasmide pET24a digéré par les mêmes enzymes puis le mélange de ligature utilisé pour transformer la souche β 2033. L'ADN plasmidique des colonies a été préparé et digéré par les enzymes *Nde*I et *Bam*HI. Ceux dont la séquence était correcte ont été utilisés pour transformer la souche BL21 (DE3)/*plys*S(Novagen). L'ADN plasmidique du mutant pSU19NLFA15T sélectionné précédemment a été préparé puis digéré par les enzymes *Nde*I et *Bam*HI. Le fragment *Nde*I -*Bam*HI correspondant a ensuite été inséré dans le plasmide pET24a digéré par les mêmes enzymes pour donner le plasmide d'expression pETLFA15T utile à l'expression de la protéine mutée. Une souche d'*E.coli* transformée à l'aide du plasmide pETLFA15T a été déposée à la CNCM le 22 mars 2004 sous le numéro d'accession I-3192. La surproduction des deux *N-*deoxyribosyltransférases, native et mutée, a été obtenue à partir de cultures de cette souche dans 500ml de milieu LB supplémenté en Km et Cm. Ces cultures ont été induites à une DO₆₀₀ =0,6 par addition d'IPTG (0,4mM), l'incubation étant poursuivie pendant 2h30 à 37°C.

Les cellules sont ensuite centrifugées 15' à 4000rpm à 4°C, lavées dans 50 ml de tampon phosphate puis le culot obtenu après centrifugation est conservé une nuit à -20°C. Le culot bactérien remis en suspension dans 20 ml de tampon phosphate est ensuite lysé par un passage à la presse de French à 14000psi. Le lysat est centrifugé pendant 90' à 50000 rpm. Le surnageant contenant les protéines solubles est ensuite précipité au sulfate d'ammonium (40 % saturation). Le précipité obtenu après centrifugation à 13900rpm (20000g) pendant 30' à 4°C est remis en suspension dans 1 ml de tampon phosphate 100mM pH7,5 NaCl 1,5M puis déposé sur une colonne gel filtration Sephacryl S200 (Amersham-Pharmacia). Les fractions sont ensuite analysées par gel SDS-PAGE et l'activité enzymatique déterminée. Les fractions les plus actives et les plus pures sont dialysées durant une nuit à 4°C contre le même tampon à pH=6,0. La concentration protéique est déterminée par mesure de la DO à 280 nm.

La mesure des activités enzymatiques est effectuée comme décrit dans le paragraphe 4.2.

### 5) Résultats

Les clones transformants de la souche d'*E.coli* PAK9, exprimant le gène *ntd* muté de *L. fermentans* ont été sélectionnés en milieu minéral glucose additionné de didésoxyuracile (ddR-U) et de cytosine (C).

Plusieurs transformants ont été obtenus et sont capables de réaliser l'échange :

ddR-Pyr + Pur ←→ ddR-Pur + Pyr ainsi que dR-Pyr + Pur ←→ dR-Pur + Pyr.

Les séquences nucléotidiques des différents variants de *ntd* sont identiques et ne diffèrent du gène sauvage que d'une mutation (indiquée en gras dans le tableau 2 ci-dessous). Dans les deux cas (*L. leichmannii* et *L. fermentum*) un acide aminé neutre (glycine et alanine) est remplacé par un acide aminé nucléophile (serine et thréonine respectivement). La conversion *N-*désoxyribosyltransférase en *N-*didésoxyribosyltransférase ou *N-*didéhydroribosyltransférase semble donc nécessiter la substitution d'un acide aminé neutre par un acide aminé nucléophile qui doit contribuer au positionnement du sucre favorisant sa catalyse. Il est remarquable de noter dans le tableau 2 que toutes les *N-*désoxyribosyltransférases ainsi qu'un certain nombre de protéines (de fonction inconnue) homologues possèdent à cette position une glycine ou une alanine.

**TABLEAU 2**

| **Origine du gène muté** | **Séquence protéique correspondante** | | |
|---|---|---|---|
| NTD *Lactobacillus. acidophilus* | MMAKTKTLYF | G | AGWFNEKQNKAYKAAMEALKQN |
| NTD *Lactobacillus. helveticus* | MNKKKTLYF | G | AGWFNEKQNKAYKEAMAALKEN |
| NTD *Lactobacillus. leichmannii* | MPKKTIYF | G | AGWFTDRQNKAYKEAMEALKEN |
| NTD LIG9S | MPKKTIYF | S | AGWFTDRQNKAYKEAMEALKEN |
| PTD *Lactobacillus. helveticus* | MKAVVPTG-KIYL | G | SPFYSDAQRERAAKAKELLAKN |
| *Lactobacillus gasseri* | MTKQKTVYF | G | AGWFTETQNKAY |
| NTD *Lactobacillus. fermentum* | LKNTDPVANTKIYL | A | TSFFNEEQRARIPQALAQLEAN |
| NTDLFA15T | LKNTDPVANTKIYL | T | TSFFNEEQRARIPQALAQLEAN |
| *Oenococcus oeni* MCW | MNMAKNIYL | A | SPFFDDEQIARVKKIEKALESN |
| *Leuconostoc mesenteroides* ATCC 8293 | KNVYL | A | SPFFDKEQIERVERVEKALAAN |
| *Lactobacillus plantarum WCFS1* | VYL | A | APFFDEAQKERIQQVKSALLAN |
| *Lactococcus lactis IL9403* | NQAVNVYL | A | APFFSESQIKK |

Les activités enzymatiques des *N-*désoxyribosyltransférases natives et mutantes de *L. leichmannii* (LL et LL G9S) et de *L. fermentum* (LF et LF A15T) dans les réactions d'échange dT +C ←→dC + T, ddT +C ←→ddC + T et d4T +C ←→d4C + T ont été évaluées à partir d'extraits bruts ou des protéines purifiées.

Les résultats reportés dans le tableau 3 ci-dessous montrent que l'activité spécifique du mutant LFA15T est inférieure à celle de l'enzyme native (LF) pour le transfert de désoxyribose mais que celle-ci est supérieure pour le transfert de didésoxyribose ou de didéhydroribose. Pour le transfert de désoxyribose, l'activité est diminuée d'un rapport de 7, tandis que celle-ci est augmentée par 3 dans le cas du transfert de didésoxyribose et par 35 dans le cas du didéhydroribose.

**TABLEAU 3**

| | LL | LL G9S | LF | LFA15T |
|---|---|---|---|---|
| dT + C | 100 | 10 | 76,5 | 10,7 |
| ddT + C | 0,2 | 2,5 | 0,9 | 2,5 |
| d4T + C | 0,5 | 8 | 2,1 | 73,5 |

| | | | | |
|---|---|---|---|---|
| Nota : 100% en haut de la colonne LL représente l'activité spécifique de l'enzyme NTD de *L. leichmannii* lors de la réaction dT + C ←→dC+ T. | | | | |

Le tableau 4 ci-dessous, montre le détail des résultats de tests d'activité enzymatique pour l'enzyme native et l'enzyme mutée de *B.fermentum* pour chacune des réactions dT + C, ddT + C et d4T + C. On retrouve dans la première colonne du tableau les valeurs de constante d'affinité (Km), dans la seconde, de vitesse maximale de réaction (Vmax), dans la troisième, de constante de catalyse (Kcat), et dans la dernière le rapport des constantes d'affinité et de catalyse (Km/Kcat) rendant compte de l'efficacité des enzymes testées. Ces différentes valeurs ont été mesurées selon le protocole décrit dans la littérature [P A Kaminski (2002) "Functional cloning, heterologous expression and purification of two different N-deoxyribosyltransferases from Lactobacillus helveticus" J. Biol. Chem; vol. 277; 14400-14407]. L'enzyme mutée selon le procédé de l'invention montre une meilleure activité catalytique sur d4T et sur ddT que l'enzyme native. Les activités sont augmentées respectivement de 60 % et 54 %. En outre, l'enzyme mutée LFA15T est 60 fois plus efficace que l'enzyme native LF dans l'échange ddT + X et 7, 5 fois plus efficace dans l'échange d4T + X.

**TABLEAU 4**

| | Km | Vmax | Kcat | Kcat/km |
|---|---|---|---|---|
| | µM | µmol/s | µmol/s/µg | |
| LF dT | 124 | 6,65 | 0,665 | 5,36.10-³ 5,36.10⁻³ |
| LF ddT | 80 | 5,7 | 0,038 | 0,047.10⁻² |
| LF d4T | 1250 | 24 | 0,56 | 0,448.10⁻³ |
| LFA15T dT | 371 | 9,7 | 0,242 | 0,65.10⁻³ |
| LFA15T ddT | 53 | 7,8 | 0,156 | 2,9.10⁻³ |
| LFA15Td4T | 1,1 | 18,4 | 3,68 | 3,34 |

L'enzyme sélectionnée trouve donc son application dans la synthèse enzymatique de 2',3'-didésoxynucléosides et de 2',3'-didésoxy, 2',3'-didéhydronucléosides de bases naturelles ddC, ddA, ddl, d4T, d4C, d4G (Ray *et al* 2002 ; Stuyver *et al,* 2002) ou modifiées (Pokrovsky *et al*, 2001 Chong *et al*, 2002) tels que (1β-3'-fluoro) 2',3'-didésoxy, 2',3'-didéhydro-4'-thio-Nucleosides comportant ou non des radioéléments.

### 6) Détermination des résidus impliqués dans le site catalytique de l'enzyme Ntd:

Comme le montre l'alignement de la figure 3, les résidus Y(Tyr)13, D (Asp)77, D (Asp) 97, E(Glu)103 et M (Met)132 (numérotation établie par rapport à Ntd de *B. fermentum -* SEQ ID No.2) se trouvent particulièrement conservés chez les protéines Ntd des différents microorganismes représentés. Des expériences de mutagenèse ponctuelle ciblant ces résidus ont permis d'établir qu'ils étaient impliqués dans le site catalytique de l'enzyme. En effet, la mutation d'un de ces résidus entraîne une perte d'activité de l'enzyme de l'ordre de 90 %.

### REFERENCES

Bartolome B, Jubete Y, Martinez E, de la Cruz F. (1991) »Construction and properties of a family of pACYC184-derived cloning vectors compatible with pBR322 and its derivatives. Gene. 102 :75-8
Carson D.A. & Wasson D.B. (1988) Synthesis of 2',3'-dideoxynucleosides by enzymatic trans-glycosylation. Biochem. Biophys. Res. Comm. 155 : 829-834.
Chong Y, Choo H, Choi Y, Mathew J, Schinazi RF, Chu CK. Stereoselective synthesis and antiviral activity of D-2',3'-didehydro-2',3'-dideoxy-2'-fluoro-4'-thionucleosides. J Med Chem. 2002 45:4888-98.
Dower WJ, Miller JF, Ragsdale CW. (1988) « High efficiency transformation of E. coli by high voltage electroporation. » Nucleic Acids Res. 16 :6127-45.
Fischer, X., Kaun, E. and Genz, ,U. (1990) 2',3'-Dideoxyribofuranosides and process for their production. Ger. Offen. DE 3840160.
Pokrovsky AG, Pronayeva TR, Fedyuk NV, Shirokova EA, Khandazhinskaya AL, Tarusova NB, Karpenko IL, Krayevsky AA. (2001) Anti-HIV activity of novel phosphonate derivatives of AZT, d4T, and ddA. Nucleosides Nucleotides Nucleic Acids. 4-7:767-9.
Ray AS, Yang Z, Chu CK, Anderson KS. Novel use of a guanosine prodrug approach to convert 2',3'-didehydro-2',3'-dideoxyguanosine into a viable antiviral agent. Antimicrob Agents Chemother. 2002 46:887-91.
Richaud C, MengiN-Lecreulx D, Pochet S, Johnson EJ, Cohen GN, Marliere P. (1993) Directed evolution of biosynthetic pathways. Recruitment of cysteine thioethers for constructing the cell wall of Escherichia coli. J Biol Chem. 268 :26827-35.
Secrist JA 3rd, Riggs RM, Tiwari KN, Montgomery JA.Synthesis and anti-HIV activity of 4'-thio-2',3'-dideoxynucleosides. J Med Chem 1992 35: 533-8
Stuyver LJ, Lostia S, Adams M, Mathew JS, Pai BS, Grier J, Thamish PM, ChoiY, Chong Y, Choo H, Chu CK, Otto MJ, Schinazi RF. Antiviral activities and cellular toxicities of modified 2',3'-dideoxy-2',3'-didehydrocytidine analogues.Antimicrob Agents Chemother. 2002 46: 3854-60.
Van Draanen NA, Freeman GA, Short SA, Harvey R, Jansen R, Szczech G, Koszalka GW. (1996) « Synthesis and antiviral activity of 2'-deoxy-4'-thio purine nucleosides. »J Med Chem 39: 538-42

## Revendications

1. Protéine ayant une activité N-didésoxyribosyltransférase présentant un pourcentage d'identité avec SEQ ID NO.4 égal ou supérieur à 70 % et comprenant un résidu thréonine correspondant au point de mutation A15T, la correspondance entre le résidu thréonine et le point de mutation A15T étant établie par l'alignement de la séquence de ladite protéine avec SEQ ID NO.4., selon la méthode Clustal W.

2. Protéine selon la revendication 1, **caractérisé en ce qu'**elle présente un pourcentage d'identité avec SEQ ID NO.4 égal ou supérieur à 80%.

3. Protéine selon la revendication 1, **caractérisé en ce qu'**elle présente un pourcentage d'identité avec SEQ ID NO.4 égal ou supérieur à 90%.

4. Protéine selon l'une quelconque des revendications 1 à 3, **caractérisé en ce qu'**elle comprend, en outre, les résidus correspondant à Y13, D77, D97, E103 et M132 de SEQ ID NO.4, la correspondance avec ces résidus étant établie par l'alignement de la séquence de ladite protéine avec SEQ ID NO.4, selon la méthode Clustal W.

5. Protéine **caractérisée en ce qu'**elle comprend la séquence SEQ ID No.4.

6. Protéine **caractérisée en ce qu'**elle consiste en la protéine LFA15T ayant pour séquence SEQ ID No.4.

7. Protéine selon l'une quelconque des revendications 1 à 6, **caractérisée en ce que** ladite protéine présente, en outre, une activité de transfert du désoxyribose et du didésoxyribose et/ou didéhydroribose.

8. Acide nucléique codant une protéine selon l'une quelconque des revendications 1 à 7.

9. Acide nucléique comprenant la séquence SEQ ID No. 3 codant la protéine LFA15T.

10. Vecteur d'expression comprenant un acide nucléique selon l'une des revendications 8 ou 9.

11. Vecteur selon la revendication 10, **caractérisé en ce que** ledit acide nucléique est fusionné à un promoteur efficace pour l'expression de la protéine dans les cellules eucaryotes et/ou procaryotes.

12. Vecteur selon l'une des revendications 10 et 11, **caractérisé en ce qu'**il s'agit d'un plasmide capable de transformer et de se maintenir chez *E. coli.*

13. Cellule hôte comprenant un vecteur selon l'une des revendications 11 et 12.

14. Utilisation *in-vitro* d'une protéine ayant une activité N-didésoxyribosyltransférase selon l'une quelconque des revendications 1 à 7 pour le transfert d'un didésoxyribose (ddR) d'un didésoxyribonucléoside sur un autre nucléoside.

15. Utilisation selon la revendication 14, dans la synthèse de 2',3'-didesoxynucléosides.

16. Utilisation selon la revendication 14, dans la synthèse de 2',3'-didéhydro-2',3'-didesoxynudéosides.

17. Utilisation selon l'une quelconque des revendications 14 à 16, pour la préparation d'analogues de nucléosides ou nucléotides possédant des propriétés antitumorales.

18. Utilisation selon la revendication 14 pour la préparation du ddl ou du ddC.

19. Procédé de préparation de composés comprenant une étape consistant à mettre en oeuvre une protéine selon l'une des revendications 1 à 7.

20. Procédé selon la revendication 19 pour la préparation d'analogues de nucléosides ou nucléotides utiles pour le traitement du cancer ou de maladies infectieuses, tels que des didésoxyribonucléosides, comme le ddC et le ddl ou des didéhydro-didesoxyribonucléosides.

21. Souche d'*E. coli* déposée à la CNCM le 22 mars 2004 sous le numéro d'accession I-3192.

## Claims

1. Protein having an N-dideoxyribosyl transferase activity, with a percentage identity with SEQ ID No. 4 equal to or greater than 70 %, and comprising a threonine residue corresponding to the mutation point A15T, the correspondance between the threonine residue and the mutation point A15T being established by alignment of the sequence of said protein with SEQ ID No.4 according to the ClustalW method.

2. Protein according to claim 1, **characterized in that** it has a percentage identity with SEQ ID No. 4 equal to or greater than 80 %.

3. Protein according to claim 1, **characterized in that** it has a percentage identity with SEQ ID No.4 equal to or greater than 90 %.

4. Protein according to any one of claims 1 to 3, **characterized in that** it moreover comprises the residues corresponding to Y13, D77, D97, E103 and M132 of SEQ ID No. 4, the correspondance with these residues being established by alignment of the sequence of said protein with SEQ ID No. 4 according to the ClustalW method.

5. Protein **characterized in that** it comprises the sequence SEQ ID No.4.

6. Protein **characterized in that** it consists of the LFA15T protein, having the sequence SEQ ID No. 4.

7. Protein according to any one of claim 1 to 6, **characterized in that** said protein moreover has a deoxyribose and dideoxyribose and/or didehydroribose transfer activity.

8. Nucleic acid coding for a protein according to any one of claims 1 to 7.

9. Nucleic acid comprising the sequence SEQ ID No.3 coding for the LFA15T protein.

10. Expression vector comprising a nucleic acid according to one of claims 8 or 9.

11. Vector according to claim 10, **characterized in that** the nucleic acid is fused to an effective promoter for the expression of the protein in the eukaryotic and/or prokaryotic cells.

12. Vector according to one of claims 10 and 11, **characterized in that** it is a plasmid capable of transforming and being maintained in *E. coli.*

13. Host cell comprising a vector according to one of claims 11 and 12.

14. *In-vitro* use of a protein having an N-dideoxyribosyl transferase activity according to any one of claims 1 to 7 for the transfer of a dideoxyribose (ddR) from a dideoxyribonucleoside to another nucleoside.

15. Use according to claim 14, in the synthesis of 2',3'-dideoxynucleosides.

16. Use according to claim 14, in the synthesis of 2',3'-didehydro-2',3'-dideoxynucleosides.

17. Use according to any one of claims 14 to 16 for the preparation of nucleoside or nucleotide analogues possessing anti-tumor properties.

18. Use according to claim 14 for the preparation of ddl or ddC.

19. Method for the preparation of compounds comprising a stage consisting of utilizing a protein according to one of claims 1 to 7.

20. Method according to claim 19 for the preparation of nucleoside or nucleotide analogues useful for the treatment of cancer or infectious diseases, such as dideoxyribonucleosides, such as ddC and ddl or didehydrodideoxyrihonucleosides.

21. Strain of *E. coli* deposited at the CNCM on 22nd March 2004 under accession number I-3192.

## Patentansprüche

1. Protein mit N-Didesoxyribosyltransferaseaktivität, das mit SEQ ID NO.4 eine Identität von 70 Prozent oder mehr aufweist und das einen Threoninrest umfasst, der mit dem Mutationspunkt A15T übereinstimmt, wobei die Übereinstimmung zwischen dem Threoninrest und dem Mutationspunkt A15T durch Alignment der Sequenz dieses Proteins mit SEQ ID NO.4 gemäß der Clustal-W-Methode festgestellt worden ist.

2. Protein nach Anspruch 1, **dadurch gekennzeichnet, dass** es eine Identität mit SEQ ID NO.4 von 80 Prozent oder mehr aufweist.

3. Protein nach Anspruch 1, **dadurch gekennzeichnet, dass** es eine Identität mit SEQ ID NO.4 von 90 Prozent oder mehr aufweist.

4. Protein nach einem der Ansprüche 1 bis 3, **dadurch gekennzeichnet, dass** es weiterhin die Reste, die mit Y13, D77, D97, E103 und M132 von SEQ ID NO.4 übereinstimmen, umfasst, wobei die Übereinstimmung mit diesen Resten durch Alignment der Sequenz dieses Proteins mit SEQ ID NO.4 nach der Clustal-W-Methode festgestellt worden ist.

5. Protein, **dadurch gekennzeichnet, dass** es die Sequenz SEQ ID NO.4 umfasst.

6. Protein, **dadurch gekennzeichnet, dass** es aus dem Protein LFA15T mit der Sequenz SEQ ID NO.4 besteht.

7. Protein nach einem der Ansprüche 1 bis 6, **dadurch gekennzeichnet, dass** das Protein weiterhin eine Desoxyribose- und Didesoxyribose- und/oder Didehydroribosetransferaktivität aufweist.

8. Nukleinsäure, die für ein Protein nach einem der Ansprüche 1 bis 7 codiert.

9. Nukleinsäure, die die Sequenz SEQ ID NO.3, welche für das Protein LFA15T codiert, umfasst.

10. Expressionsvektor, der eine Nukleinsäure nach einem der Ansprüche 8 oder 9 umfasst.

11. Vektor nach Anspruch 10, **dadurch gekennzeichnet, dass** diese Nukleinsäure mit einem wirksamen Promoter für die Expression des Proteins in eukaryontischen und/oder prokaryontischen Zellen fusioniert ist.

12. Vektor nach einem der Ansprüche 10 und 11, **dadurch gekennzeichnet, dass** es sich um ein Plasmid handelt, das fähig ist, *E. coli* zu transformieren und in *E. coli* erhalten zu bleiben.

13. Wirtszelle, die einen Vektor nach einem der Ansprüche 11 und 12 umfasst.

14. In-vitro-Verwendung eines Proteins mit einer N-Didesoxyribosyltransferaseaktivität nach einem der Ansprüche 1 bis 7 für den Transfer einer Didesoxyribose (ddR) eines Didesoxyribonukleosids auf ein anderes Nukleosid.

15. Verwendung nach Anspruch 14 in der Synthese von 2',3'-Didesoxynukleosiden.

16. Verwendung nach Anspruch 14 in der Synthese von 2',3'-Didehydro-2',3'-didesoxynukleosiden.

17. Verwendung nach einem der Ansprüche 14 bis 16, für die Herstellung von Nukleosid- oder Nukleotidanalogen mit Antitumoreigenschaften.

18. Verwendung nach Anspruch 14 für die Herstellung von ddI oder ddC.

19. Verfahren zur Herstellung von Verbindungen, umfassend einen Schritt, der darin besteht, das man ein Protein nach einem der Ansprüche 1 bis 7 einsetzt.

20. Verfahren nach Anspruch 19 für die Herstellung von Nukleosid- oder Nukleotidanalogen, die sich für die Behandlung von Krebs oder Infektionskrankheiten eignen, wie Didesoxyribonukleoside wie ddC und ddI oder Didehydrodidesoxyribonukleoside.

21. *E. coli*-Stamm, der am 22. März 2004 unter der Zugangsnummer 1-3192 bei der CNCM hinterlegt worden ist.


### SEQUENCE LISTING

<110> INSTITUT PASTEUR
<120> N désoxyribosyltransférases de Lactobacillus fermentum et application à la synthèse enzymatique de 2',3' didésoxynucléosides et de 2',3' didéhydronucléosides
<130> BIF 116236 FR
<160> 12
<170> PatentIn version 3.1
<210> 1
   <211> 504
   <212> DNA
   <213> Lactobacillus fermentum filed at Genbank accession N° AY064168
<400> 1
   atgaaaaata ccgacccagt tgctaacact aaaatttacc tggctaccag cttcttcaac      60
   gaagaacaac gtgcccgcat ccctcaagct ctagcccaac tagaagccaa cccgactgtc     120
   ggcgttgttc accagccatt cgatttccaa tataaagatg cacgcgtaga ctccgatcct     180
   gccggcgtct ttggcagcct cgaatggcaa attgccactt acaataacga cctcaacgcg     240
   gtaggaactt ccgatgtctg cgttgcttta tacgatatgg accaaattga cgaaggaatt     300
   tgtatggaaa tcggcatgtt cgtcgccctc cataaaccta tcgttttact accttttact     360
   aagaaagata agtctgctta tgaagctaac ctaatgctag cacggggtgt aactacctgg     420
   ttggaaccta atgactttag tcccttaaaa gactttaact ttaaccaccc aatggctcaa     480
   cctttcccac cattcaaggt tttc                                            504
<210> 2
   <211> 168
   <212> PRT
   <213> Lactobacillus fermentum
<400> 2
   Met Lys Asn Thr Asp Pro Val Ala Asn Thr Lys Ile Tyr Leu Ala Thr
   1               5                   10                  15
   Ser Phe Phe Asn Glu Glu Gln Arg Ala Arg Ile Pro Gln Ala Leu Ala
               20                  25                  30
   Gln Leu Glu Ala Asn Pro Thr Val Gly Val Val His Gln Pro Phe Asp
           35                  40                  45
   Phe Gln Tyr Lys Asp Ala Arg Val Asp Ser Asp Pro Ala Gly Val Phe
   50 55 60
   Gly Ser Leu Glu Trp Gln Ile Ala Thr Tyr Asn Asn Asp Leu Asn Ala
   65 70 75 80
   Val Gly Thr Ser Asp Val Cys Val Ala Leu Tyr Asp Met Asp Gln Ile
   85 90 95
   Asp Glu Gly Ile Cys Met Glu Ile Gly Met Phe Val Ala Leu His Lys
   100 105 110
   Pro Ile Val Leu Leu Pro Phe Thr Lys Lys Asp Lys Ser Ala Tyr Glu
   115 120 125
   Ala Asn Leu Met Leu Ala Arg Gly Val Thr Thr Trp Leu Glu Pro Asn
   130 135 140
   Asp Phe Ser Pro Leu Lys Asp Phe Asn Phe Asn His Pro Met Ala Gln
   145 150 155 160
   Pro Phe Pro Pro Phe Lys Val Phe
   165
<210> 3
   <211> 504
   <212> DNA
   <213> Lactobacillus fermentum
<400> 3
   atgaaaaata ccgacccagt tgctaacact aaaatttacc tgactaccag cttcttcaac 60
   gaagaacaac gtgcccgcat ccctcaagct ctagcccaac tagaagccaa cccgactgtc 120
   ggcgttgttc accagccatt cgatttccaa tataaagatg cacgcgtaga ctccgatcct 180
   gccggcgtct ttggcagcct cgaatggcaa attgccactt acaataacga cctcaacgcg 240
   gtaggaactt ccgatgtctg cgttgcttta tacgatatgg accaaattga cgaaggaatt 300
   tgtatggaaa tcggcatgtt cgtcgccctc cataaaccta tcgttttact accttttact 360
   aagaaagata agtctgctta tgaagctaac ctaatgctag cacggggtgt aactacctgg 420
   ttggaaccta atgactttag tcccttaaaa gactttaact ttaaccaccc aatggctcaa 480
   cctttcccac cattcaaggt tttc 504
<210> 4
   <211> 168
   <212> PRT
   <213> Lactobacillus fermentum A15T
<400> 4
   Met Lys Asn Thr Asp Pro Val Ala Asn Thr Lys Ile Tyr Leu Thr Thr
   1               5                   10                  15
   Ser Phe Phe Asn Glu Glu Gln Arg Ala Arg Ile Pro Gln Ala Leu Ala
               20                  25                  30
   Gln Leu Glu Ala Asn Pro Thr Val Gly Val Val His Gln Pro Phe Asp
           35                  40                  45
   Phe Gln Tyr Lys Asp Ala Arg Val Asp Ser Asp Pro Ala Gly Val Phe
       50                  55                  60
   Gly Ser Leu Glu Trp Gln Ile Ala Thr Tyr Asn Asn Asp Leu Asn Ala
   65                  70                  75                  80
   Val Gly Thr Ser Asp Val Cys Val Ala Leu Tyr Asp Met Asp Gln Ile
                   85                  90                  95
   Asp Glu Gly Ile Cys Met Glu Ile Gly Met Phe Val Ala Leu His Lys
               100                 105                 110
   Pro Ile Val Leu Leu Pro Phe Thr Lys Lys Asp Lys Ser Ala Tyr Glu
           115                 120                 125
   Ala Asn Leu Met Leu Ala Arg Gly Val Thr Thr Trp Leu Glu Pro Asn
       130                 135                 140
   Asp Phe Ser Pro Leu Lys Asp Phe Asn Phe Asn His Pro Met Ala Gln
   145                 150                 155                 160
   Pro Phe Pro Pro Phe Lys Val Phe
                   165
<210> 5
   <211> 39
   <212> DNA
   <213> Artificial
<220>
   <223> amorce
<400> 5
   caatttcaca caggaaacac atatgaccat gattacgcc 39
<210> 6
   <211> 31
   <212> DNA
   <213> Artificial
<220>
   <223> amorce
<400> 6
   tgtttcctgt gtgaaattgt tatccgctca c 31
<210> 7
   <211> 32
   <212> DNA
   <213> Artificial
<220>
   <223> amorce
<400> 7
   gatatacata tgaaaaatac cgacccagtt gc 32
<210> 8
   <211> 39
   <212> DNA
   <213> Artificial
<220>
   <223> amorce
<220>
   <221> misc_feature
   <223> n est un nucléotide comportant une base A, T, C ou G
<220>
   <221> misc_feature
   <222> (1)..(2)
   <223> n est un nucléotide comportant une base A, T, C ou G
<400> 8
   nnggatcctt aggttagtta gaaaaccttg aatggtggg 39
<210> 9
   <211> 22
   <212> DNA
   <213> Artificial
<220>
   <223> amorce
<400> 9
   ttaatacgac tcactatagg gg 22
<210> 10
   <211> 19
   <212> DNA
   <213> Artificial
<220>
   <223> amorce
<400> 10
   gctagttatt gctcagcgg 19
<210> 11
   <211> 33
   <212> DNA
   <213> Artificial
<220>
   <223> amorce
<220>
   <221> misc_feature
   <222> (1)..(1)
   <223> n est un nucléotide comportant une base A, T, C ou G
<400> 11
   ngatatacat atgaaaaata ccgacccagt tgc 33
<210> 12
   <211> 39
   <212> DNA
   <213> Artificial
<220>
   <223> amorce
<220>
   <221> misc_feature
   <222> (1)..(2)
   <223> n est un nucléotide comportant une base A, T, C ou G
<400> 12
   nnggatcctt aggttagtta gaaaaccttg aatggtggg 39